# EUROPEAN PATENT APPLICATION

(11) **EP 0 742 029 A1**
(43) Date of publication of application: **13.11.1996**
(21) Application number: 95106808.9
(22) Date of filing: 05.05.1995
(51) Int. Cl.: A61M 25/00, A61M 25/10

(54) **Interventional catheter**

(71) Applicant: SCHNEIDER (EUROPE) AG, CH-8180 Bülach (CH)
(72) Inventor: Heller, Mathias, CH-8400 Winterthur (CH); Fehse, Jan, CH-9305 Berg (CH)
(74) Representative: Misrachi, Alfred

(57) **Abstract**

An interventional catheter for angioplasty and the like, comprising a catheter tube (1) formed of two superposed layers 3 and 4 of materials different from one another. Over the distal end 2 of the catheter tube 1 is positioned a balloon 6 with a distal neck 7 sealingly surrounding the outer layer 4 of the catheter tube. A tip 8 is formed by a piece of material 12 welded to the distal end 2 of the catheter tube and the distal neck 7 of the balloon 6 also welds to the proximal portion 9 of the tip 8.

## Description

This invention relates to an interventional catheter comprising a catheter tube having proximal and distal ends, an inner layer of material forming a longitudinal lumen in the catheter tube for the sliding fit of a guide wire, an outer layer of material superposed to said inner layer and forming an outer surface of the catheter tube, said inner and outer layers of materials being secured in relation to one another and having mechanical properties differing from one another, a balloon with proximal and distal necks whereby the distal neck of the balloon sealingly surrounds the catheter tube, and a tip at the distal end of the catheter tube.

Over the wire catheters are now widely used for interventions such as percutaneous transluminal angioplasty. A problem with these catheters is that the guide wire may clog into the longitudinal lumen of the catheter; as a result, the guide wire may follow the balloon upon withdrawal thereof after the inflation procedure, thereby making it necessary to re-insert the guide wire into the treated area of the blood vessel for re-positioning a balloon in case a second inflation is needed. The catheter also has to achieve an acceptable compromise between the requirements of some stiffness to assure good pushability and of some flexibility to assure kink resistance. And of course it has to permit safe attachment of the balloon to the catheter tube.

European Patent Application N° 93117403.1 filed October 27, 1993 by the Applicant shows a catheter comprising a catheter tube having two superposed layers of materials secured in relation to one another and with mechanical properties differing from one another, a longitudinal lumen in the catheter tube for the sliding fit of a guide wire, and a balloon with a proximal end and a distal end sealingly surrounding the catheter tube, whereby the catheter tube has an inner layer forming the longitudinal lumen and an outer layer forming the outer surface of the catheter tube. In this catheter, the inner layer is formed of a material with lower friction coefficient than the material forming the outer layer, whereby there is no more risk of having the guide wire clogging in the longitudinal lumen of the catheter tube.

The document WO 92/11893 describes an intra aortic balloon apparatus comprising a hollow catheter in which is located an elongated member forming a central lumen extending out of the catheter at the distal end thereof. An aortic pumping balloon is positioned over the elongated member and the distal end of the balloon is bonded to a tip engaged over the distal end of the elongated member. In order to achieve a balance of flexibility and remains and to avoid kinking, the elongated member is formed of an inner layer comprised of a soft elastomeric material and the outer layer is comprised of a hard plastic material. This balloon apparatus cannot be loaded with a guide wire and moved into tortuous vessels with the guide wire loaded inside the elongated tube. Furthermore, this balloon apparatus cannot be introduced into narrow vessels or stenoses nor can it be passed through narrow punctures to enter the blood vessels because of the large profile of the folded balloon resulting from the fact that the balloon is bonded to an intermediate tip which in turn is mounted over and bonded to the elongated element.

The purpose of this invention is to present an interventional, low profile, highly resistant balloon catheter with improved moveability into tortuous vessels and an added capacity of reducing the risk of injury.

To this effect, the interventional catheter according to the invention complies with the definitions given in the claims.

Accordingly, where the tip is a piece of material having proximal and distal portions whereby said proximal portion is in longitudinal extension of the outer layer, with the inner layer ending proximally of the said proximal portion and the piece of material having an inner wall in longitudinal alignment with the longitudinal lumen, the tip allows a low profile with selective flexibility at the distal end of the catheter tube; this results in a better trackability through narrow or tortuous vessels, as well as in a strong reduction of the risk of injury. There is also a superior protection of the distal extremity of the superposed layers of materials against any risk of delamination. The catheter thus conforms easily with the vessel configurations, more particularly along low radius curves, and the resulting low friction substantially reduces the risk of injury to the vessel in these delicate areas.

Where the piece of material forming the tip tapers distally, the catheter end may have a strong diameter reduction which cannot be achieved by a simple multilayer catheter end because of the hazardous loss of resistance of the superposed layers. In addition, the distal end of the catheter has an added stepped flexibility which enhances the catheter capacity to reach very narrow or tortuous vessel locations with still less risk of injury.

When the piece of material forming the tip is welded to the distal end of the catheter tube a simple and rapid manufacture may be achieved in which the distal neck of the balloon, the superposed layers of materials and tip are all weld assembled in one single step.

When the piece of material forming the tip is formed by the outer layer of the catheter tube, the outer layer may easily have a diameter reduction which simultaneously protects the inner layer of the catheter tube.

For a smooth transition and added safety, the distal neck of the balloon may sealingly surround a part of the proximal portion of the piece of material, sealing being for instance by welding.

And the piece of material forming the tip may also be formed by the distal neck of the balloon, in which case the maximal diameter reduction may be achieved without removal of material at that level while simultaneously retaining the advantage of protection of the ends of the superposed layer of the catheter tube.

These and other objects, features and advantages of the invention will become readily apparent from the following detailed description with reference to the accompanying drawings which show, diagrammatically and by way of example only, preferred but still illustrative embodiments of the invention.

Figure 1 is a longitudinal cut out of a first embodiment.

Figure 2 is a longitudinal cut out of a second embodiment.

Figure 3 is a longitudinal cut out of a third embodiment.

Figure 4 is a longitudinal cut out of a fourth embodiment.

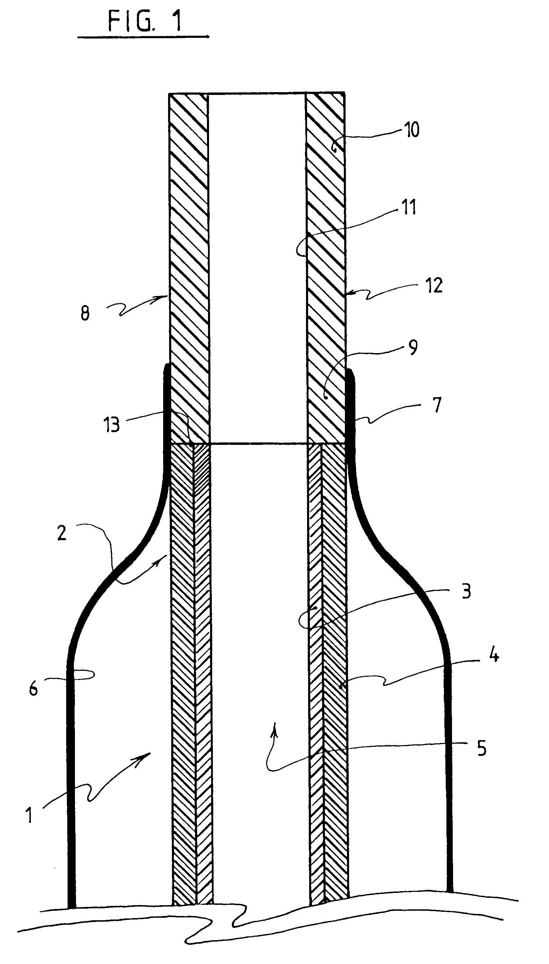

The interventional catheter shown in Figure 1 comprises a catheter tube 1 having a proximal end (not shown) and a distal end 2. The catheter tube 1 is formed of two superposed continuous layers 3 and 4 extending all over the length of tube 1; this tubing, which may be achieved by the known coextrusion technology, i.e. by extruding the outer layer 4 over the inner layer, is comprised of a polyethylene, preferably a high density polyethylene, for the inner layer 3, and of a polyamid for the outer layer 4. The inner layer thus forms a longitudinal lumen 5 with a very low friction coefficient, lower than that of the material forming the outer layer 4, and a non kinking capacity. The outer layer 4, which forms an outer surface of the catheter tube, is easily weldable to the materials commonly used for making balloons for angioplasty purposes and the like. The lumen 5 is for the sliding fit of a guide wire (not shown).

Over the distal end 2 of the catheter tube 1 is positioned a balloon 6 with a distal neck 7 which is sealed to the outer layer 4 of the catheter tube 1, for instance by welding. The balloon 6 has a proximal neck (not shown) which is affixed to the catheter tube 1 which is also provided with an inflation lumen for the balloon 6 in a conventional manner. Preferably, the balloon 6 is made of a polyamid.

A tip 8 formed by a piece of material 12 having a proximal portion 9 and a distal portion 10 is arranged at the distal end 2 of the catheter tube 1. This tip has its proximal portion 9 in longitudinal extension of the outer layer 4 of the catheter tube 1, and the inner layer 3 of the catheter tube 1 ends proximally of the proximal portion 9 of tip 8. The tip 8 has an inner wall 11 in longitudinal alignment with the longitudinal lumen 5 and therefore the proximal extremity of its proximal end fully protects the distal ends of inner and outer layers 3 and 4. The proximal end of the proximal portion 9 of tip 8 is welded to the distal end 2 of the catheter tube 1, and the distal neck 7 of the balloon 6 sealingly surrounds, for instance by welding, a part of the proximal portion 9 of tip 8.

The tip 8 is preferably made of a polyamid, and welding of the distal neck 7 of the balloon 6 to the distal end 2 of the catheter tube 1 and to the proximal portion 9 of the tip 8 may be achieved in one single step. Welding may be effected by laser or hot air.

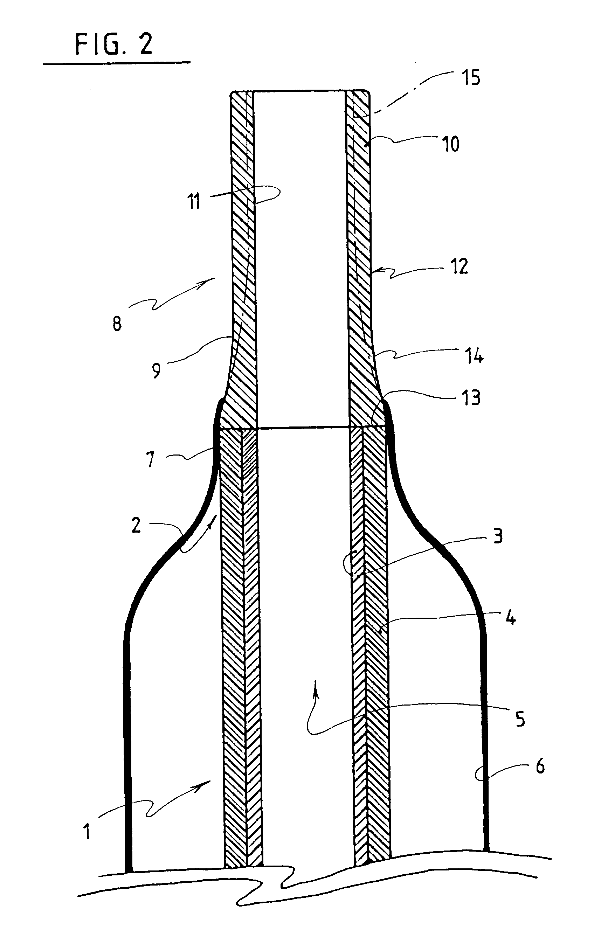

The interventional catheter shown in Figure 2 comprises the same integers and arrangements as the catheter shown in Figure 1, which are identically referenced. In addition, the piece of material 12 forming the tip 8 tapers distally as shown by reference numeral 14. As shown, the diameter reduction extends slightly above the diameter of the inner layer 3, a reduction which could not be achieved on the two layer catheter tube 1 without a serious risk of damage to the outer layer 4. It may be noted that, due to the one integral configuration of the piece of material 12 forming the tip 8, an even greater diameter reduction could be achieved, substantially beneath the diameter of the outer layer, as shown in phantom at 14.

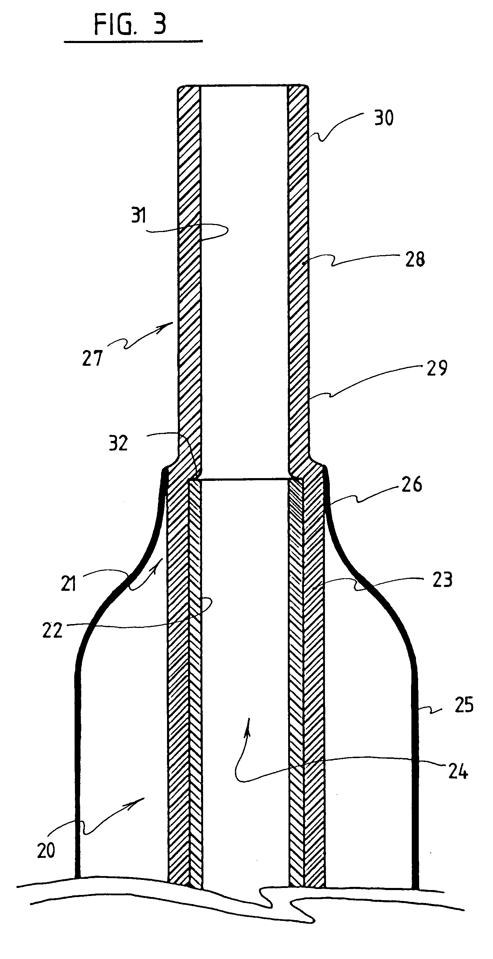

The catheter shown in Figure 3 also comprises a catheter tube 20 having a proximal end (not shown) and a distal end 21. The catheter tube 20 is formed of two superposed continuous layers 22 and 23 extending all over the length of the catheter tube with the inner layer forming a longitudinal lumen 24; this tubing may also be achieved by the coextrusion technology, the outer layer 23 being extruded over the inner layer 22. As for the examples of Figures 1 and 2, the inner layer 22 is made of a polyethylene, preferably a high density polyethylene, and the outer layer a polyamid.

Over the distal end 21 of the catheter tube 20 is arranged a balloon 25 with a distal neck 26 sealed to the outer layer 23 of the catheter tube 20. The balloon 25 has a proximal neck (not shown)which is affixed to the catheter tube 20 which is also provided with the conventional lumen for fluid entry into the balloon. The balloon 25 is also preferably made of a polyamid.

A tip 27 comprising a piece of material 28 having a proximal portion 29 and a distal portion 30 is arranged at the distal end 21 of the catheter tube 20. The piece of material 28 is formed by the outer layer 23 of catheter tube 20 which is longitudinally and distally extended beyond the distal end of inner layer 22 and constricted so as to have an inner wall 31 in longitudinal alignment with the longitudinal lumen 24 of catheter tube 20, thereby having a proximal extremity 32 fully protecting the distal end of inner layer 22. The constriction of outer layer 23 may be obtained by the shrink tube technology, i.e., by heating the piece of material 28 to have it shrink down on a mandrel of appropriate diameter.

As for the previous examples, the inner layer 22 is made of a polyethylene or high density polyethylene, whereas the outer layer 23 and the balloon are made of a polyamid.

In this embodiment, the tip 27 can also taper distally as in the embodiment of Figure 2.

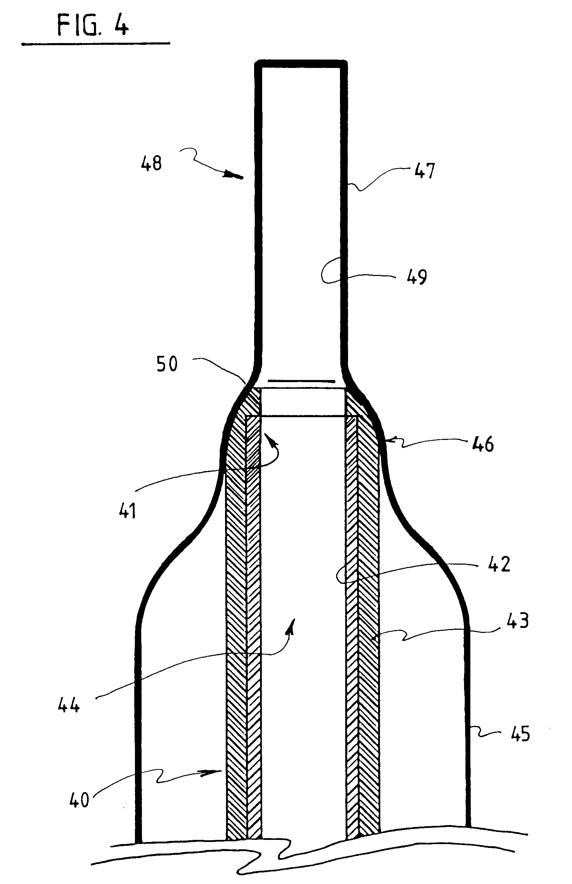

The embodiment of Figure 4 comprises a catheter tube 40 having a proximal end (not shown) and a distal end 41. The catheter tube 40 is formed of two superposed coextruded layers 42 and 43 extending all over the length of the catheter tube, with the inner layer forming a longitudinal lumen 44 for the sliding fit of a guide wire (not shown). As for the previous examples, the inner layer 42 is preferably made of a polyethylene or high density polyethylene and the outer layer a polyamid.

Over the distal end 41 of the catheter tube 40 is arranged a balloon 45, preferably made of a polyamid, having a distal neck 46 sealed to the outer layer 43. The distal neck 46 of the balloon 45 is further extended distally of the distal ends of inner and outer layers 42 and 43 to form a piece of material 47 constituting the tip 48. The extended balloon neck forming the piece of material 47, the proximal portion 50 of which is in longitudinal extension of the outer layer, is constricted so as to have an inner wall 49 in longitudinal alignment with the longitudinal lumen 44 of the catheter tube 40. The distal neck 46 of the balloon, which may be also obtained by the shrink tube technology as outlined hereinbefore, thus protects the distal ends of inner and outer layers 42 and 43.

The balloon 45 also has a proximal neck (not shown) affixed to the catheter tube which also has the necessary inflation lumen for the balloon.

## Claims

1. An interventional catheter comprising a catheter tube (1, 20, 40) having proximal and distal ends, an inner layer of material (3, 22, 42) forming a longitudinal lumen (5, 24, 44) in the catheter tube for the sliding fit of a guide wire, an outer layer of material (4, 23, 43) superposed to said inner layer and forming an outer surface of the catheter tube, said inner and outer layers of material being secured in relation to one another and having mechanical properties differing from one another, a balloon (6, 25, 45) with proximal and distal necks whereby the distal neck (7, 26, 46) of the balloon sealingly surrounds the catheter tube, and a tip (8, 27, 48) at the distal end of the catheter tube,
characterized in that the tip (8, 27, 48) is a piece of material (12, 28, 47) having proximal and distal portions whereby said proximal portion (9, 29, 50) is in longitudinal extension of said outer layer (4, 23, 43), wherein said inner layer (3, 22, 42) ends proximally of said proximal portion (9, 29, 50), and wherein said piece of material (12, 28, 47) has an inner wall (11, 31, 49) in longitudinal alignment with said longitudinal lumen (5, 24, 44).

2. An interventional catheter according to claim 1, wherein said piece of material (14, 15) tapers distally.

3. An interventional catheter according to claim 1 or 2, wherein said piece of material (12) is welded to the distal end of the catheter tube.

4. An interventional catheter according to claim 2, wherein said piece of material (28) is formed by the outer layer (23) of the catheter tube (20).

5. An interventional catheter according to any preceding claim, wherein the distal neck (7, 26) of the balloon (6, 25) sealingly surrounds a part of the proximal portion (9, 29) of the piece of material (12, 28).

6. An interventional catheter according to claim 5, wherein the distal neck (7, 26) of the balloon (6, 25) is welded to the proximal portion (9, 29) of the piece of material (12, 28).

7. An interventional catheter according to claim 2, wherein said piece of material (47) is formed by the distal neck (46) of the balloon (45).

8. An interventional catheter according to any preceding claim, wherein said piece of material (12, 28, 47) is made of a polyamid.
